# EUROPÄISCHE PATENTSCHRIFT

(11) **EP 1 221 911 B1**
(45) Veröffentlichungstag und Bekanntmachung des Hinweises auf die Patenterteilung: **13.01.2010**
(21) Anmeldenummer: 00972750.4
(22) Anmeldetag: 12.10.2000
(51) Int. Cl.: A61F 2/30, A61F 2/46, A61B 17/16, A61L 27/00

(54) **SYSTEM ZUR DECKUNG VON KNORPELDEFEKTEN MITTELS KNORPELERSATZSTRUKTUREN**
SYSTEM FOR CORRECTING CARTILAGE DEFECTS USING A CARTILAGE SUBSTITUTE STRUCTURE
SYSTEME POUR LA REPARATION DE DEFAUTS DE CARTILAGE AU MOYEN DE STRUCTURES DE REMPLACEMENT DE CARTILAGE

(30) Priorität: 20.10.1999 DE 19950406
(43) Veröffentlichungstag der Anmeldung: 17.07.2002
(73) Patentinhaber: Jansson, Volkmar, Dr., D-82205 Gilching (DE)
(72) Erfinder: Jansson, Volkmar, Dr., D-82205 Gilching (DE)
(74) Vertreter: Czybulka, Uwe
(86) Internationale Anmeldenummer: PCT/EP2000/010062
(87) Internationale Veröffentlichungsnummer: WO 2001/030276

(56) Entgegenhaltungen:
- EP-A- 0 201 651
- WO-A-96/23459
- WO-A-96/24310
- WO-A-98/56317
- DE-A- 2 933 174
- DE-A- 3 516 743
- DE-A- 19 648 876
- DE-A- 19 721 661
- DE-A- 19 803 673
- FR-A- 2 242 068
- GB-A- 2 137 209
- US-A- 5 067 964
- US-A- 5 549 704
- US-A- 5 683 465
- US-A- 5 683 466
- US-A- 5 871 540

## Beschreibung

Die Erfindung betrifft ein System zur Deckung von Knorpeldefekten.

Die Deckung von Knochen-/Knorpeldefekten stellt eine der großen Herausforderungen in der Orthopädie dar. Es gilt das Paradigma, dass die Zerstörung der hyalinen Gelenkschicht irreversibel, die "restitutio ad integrum" nicht mehr möglich ist (Geneser F (1990) Knorpelgewebe. In: Histologie. Deutscher Ärzte-Verlag Köln, S 215-216). Die nach der Zerstörung der hyalinen Gelenkschicht bestenfalls entstehenden Ersatzgewebe sind als minderwertig anzusehen. Dabei können Zerstörungen des Gelenkknorpels lokal begrenzt bleiben (z.B. Osteochondrosis dissecans) oder aber - wie bei der manifesten Gelenksarthrose - das gesamte Gelenk betreffen. Häufige Folgeschäden von Sportverletzungen oder aber auch der beginnenden Gelenksarthrose sind jedoch lokalisierte Knorpeldefekte, bei denen der darunter befindliche Knochen zwar oft bereits frei liegt, in seiner Kontinuität und Oberflächengeometrie jedoch noch weitgehend erhalten ist.

Zur Behandlungen derartiger Knochen-/Knorpeldefekte sind in der Vergangenheit eine Reihe von Behandlungskonzepten entwickelt worden. So können z.B. durch einfache Anbohrungen (z.B. "Pridie-Bohrungen") oder feine Aufmeißelungen des Knochenlagers ("microfracture") Einblutungen in die Defektzonen erzeugt werden, in denen dann ein Bindegewebe entsteht, welches als Ersatzregenerat einen gewissen - wenn auch minderwertigen - Knorpelersatz darstellt.

Neuere Verfahren sehen vor, Knorpeldefekte durch externe Züchtung ("in vitro") von Knorpelzellen zu decken ("Knorpelzelltransplantation"). Dabei werden patienteneigene Knorpelzellen entnommen, in einer Zellkultur vermehrt und anschließend in einer zweiten Operation in die Knorpeldefekte verbracht, indem sie dort unter einen über dem Defekt vernähten Periostlappen injiziert werden.

Anstatt die Zellen als freie Zellen unter einen Periostlappen einzubringen, können die Knorpelzellen auch in geeigneten Vliesstrukturen in vitro vermehrt werden (Vacanti ChA, Upton J (1994) Tissue-engineered morphogenesis of cartilage and bone by means of cell transplantation using synthetic biodegradable polymer matrices. Clin Plast Surg 21(3): 445-462). Ziel ist es dabei, die entstehenden Konstrukte nach entsprechender Zellvermehrung in den Knochen-/Knorpeldefekt zu verbringen und dort zu fixieren. Dabei werden zur Fixierung häufig biologische Kleber (z.B. Fibrinkleber) verwandt. Aber natürlich ist auch die Fixierung mit resorbierbaren Schrauben, Stiften, Dübeln o.ä. möglich.

Eine Übersicht und kritische Beleuchtung der diskutierten Verfahren zur Knorpelzelltransplantation findet sich in: Mesmer K, Gillquist J (1996) Cartilage Repair, a critical review. Acta Orthop Scand. 67(5):523529.

In DE 197 21 661 A1 wird ein Konstrukt angegeben, bei dem das kritische Problem der knöchernen Verankerung eines Knorpelersatzkonstruktes im Sinne einer "Knochen- und KnorpelErsatzstruktur" gelöst wird. Hier wird die "Knorpelersatzstruktur (z.B. Vlies) mit einer "Knochenersatzstruktur" fest verbunden, so dass das Gewebe aus dem Knochenmark in beide Schichten penetrieren und sich unter dem mechanischen Druck zu Knorpel- und Knochenzellen differenzieren kann. Mit diesem Verfahren können nicht nur große Knorpel-, sondern insbesondere auch große den Knorpeldefekt begleitende Knochendefekte versorgt werden.

Auch in DE 198 03 673 A1 wird ein Lösungsansatz vorgeschlagen, bei dem die Knochenersatzstruktur als ein "Mittel zur Förderung der knöchernen Integration" bezeichnet wird. Auch hier wird durch Ankopplung der Knorpelersatzstruktur an eine in den Knochen ragende Unterschicht eine Verbindung der Knorpelersatzstruktur mit dem Knochen gesucht.

Trotz der vielfältigen Bemühungen sind die aus dem Stand der Technik bekannten Knorpelersatzstrukturen - und insbesondere deren Verbindung mit dem Knochen - mit einer Reihe von Nachteilen behaftet.

Bereits in DE 197 21 661 A1 und DE 198 03 673 A1 wird auf die Problematik der Fixierung im Knochenlager hingewiesen. Beide Lösungsvorschläge sehen die Entfernung der obersten Knochenschicht vor, um eine Verankerung im subchondralen Knochen mit einer Knochenersatzstruktur (DE 197 21 661 A1) bzw. einer "Integrationsschicht", d. h. einem "Mittel zur Förderung der knöchernen Integration" (DE 198 03 673 A1) vorzunehmen. Zusätzlich wird in DE 198 03 673 A1 die Fixierung der (auch schon in DE 197 21 661 A1 angegebenen) Gelenkabschlussmembran (bei DE 198 03 673 A1 "Deckfolie" genannt) auf der Knorpelersatzstruktur (bei DE 198 03 673 A1 "poröse Träger" genannt) mit Nahtmaterial oder Stiften angegeben. Eine Fixierung des gesamten Konstruktes im Knochenlager zur weiteren Verstärkung der Haftung der angegebenen Integrationsschicht (z.B. mit Dübeln, Stiften Schrauben etc.) findet sich nicht. Trotzdem muss bei beiden Verfahren die unter der zu ersetzenden Knorpelschicht befindliche Knochenschicht entweder zur Aufnahme der Knochenersatzstruktur (bzw. Integrationschicht) entfernt werden, oder aber diese Knochenschicht ist aufgrund eines ausgeprägten Knochen-/Knorpeldefektes ohnehin nicht mehr vorhanden.

In vielen Fällen ist aber die Entfernung des unter dem geschädigten Knorpel liegenden Knochens gar nicht erforderlich, da der Knorpelschaden den Knochen oft gar nicht oder nur unwesentlich erfasst. Von erheblichem Nachteil wäre es, wenn in diesen Fällen ein Teil des ja noch intakten Knochens zur Fixierung der Knorpelersatzstruktur entfernt werden muss, insbesondere, wenn dadurch die Kontur der Gelenkoberfläche verloren geht. Im Gegenteil ist der Erhalt der äußeren knöchernen Gelenkkontur (bzw. deren Rekonstruktion wie in DE 197 21 661 A1 angebeben) wichtig, da nur so letztendlich auch eine geometrisch korrekte Gelenkknorpelfläche entstehen kann. In den Fällen, in denen die unter einem reinen Knorpeldefekt in vielen Fällen noch weitgehend intakte Knochenschicht aus den genannten Gründen ***nicht*** entfernt werden soll, ist die Verankerung mit einer Knochenersatzstruktur (DE 197 21 661 A1) bzw. Integrationsschicht (DE 198 03 673 A1) nicht möglich oder nur schwer praktikabel. In beiden Fällen muss für die Knochenersatzstruktur und Integrationsschicht ein entsprechend großer Raum im Knochen geschaffen werden, damit eine stabile Verankerung des Konstruktes gegeben ist. Die alleinige Auflage auf die Knochenoberfläche dürfte in beiden Fällen - auch mit einer als "Haftvermittler" eingesetzten Integrationsschicht -zu keiner stabilen Fixierung führen. Auch trägt eine auf die intakte Knochenoberfläche aufgebrachte Integrationsschicht entsprechend ihrer Dicke auf und verhindert so die wichtige Kongruenz der Knorpelersatzschicht zu der umliegenden Gelenkfläche ("Stufenbildung").

Auch ist eine zwischen Knochen und Knorpelersatzstruktur eingebrachte Zwischenschicht in den Fällen problematisch, in denen ein Einwandern der aus dem Knochenmark stammenden Stammzellen (die sich in Knorpelzellen differenzieren können und dann Knorpelvorläuferzellen entsprechen) gewollt wird. Das Prinzip der Umdifferenzierung mesenchymalen Gewebes ist in DE 197 21 661 A1 ausführlich beschrieben. In diesen Fällen ist speziell darauf zu achten, dass eine ***offene*** Verbindung zwischen dem Knochenlager einerseits und der Knorpelersatzstruktur andererseits besteht, damit eine Migration der Zellen aus dem Knochen in die Knorpelersatzstruktur stattfinden kann. Jede Art der Zwischenschicht, die eine solche Migration der Zellen behindert, wie das z.B. bei einem Haftvermittler wie der in DE 198 03 673 A1 genannten Integrationsschicht der Fall ist, ist als ungünstig anzusehen. Im Gegenteil muss in den Fällen, bei denen eine Migration der aus dem Knochenmark stammenden Stammzellen in die Knorpelersatzstruktur gewünscht wird, die subchondrale Skleroseschicht an einigen Stellen lokalisiert eröffnet werden (ohne dass die Kontur der Knochenoberfläche insgesamt verloren geht), so dass Zellen aus dem Knochenmark austreten und in die Knorpelersatzstruktur eindringen können.

Die aus der Technik und auch in der Medizin - z.B. zur Anheftung von Meniskusrissen - bekannten Schraub-, Stift- oder ähnliche Verbindungselemente sind problematisch: Da derartige Verbindungselemente systembedingt durch die Knorpelersatzstruktur hindurch in das Knochenlager eingebracht werden müssen, verbleibt ein Teil eines solchen Verbindungselementes (z.B. Schraubenkopf) auf der dem Gelenk zugewandten Seite. Da die Verankerungselemente naturgemäß - im Vergleich zur Knorpelersatzstruktur und dem gegenüber liegenden Gelenkknorpel der korrespondierenden Gelenkfläche - sehr hart sind, würden derartige die Knorpelersatzstruktur unterbrechende Strukturen den gegenüber liegenden Gelenkknorpel mechanisch schädigen. Des Weiteren wäre im Bereich der Verankerungselemente die Knorpelersatzstruktur - und damit die neue Gelenkfläche - unterbrochen und würde entsprechende Defekte aufweisen.

Aus der DE-A-2933174 ist eine Struktur zur Deckung von Knorpeldefekten mittels einer Knorpelersatzstruktur bekannt, die im Bereich des Knorpeldefektes einsetzbar ist, wobei die Knorpelersatzstruktur an ihrer dem Knochen zugewandten Seite fest mit Verankerungselementen verbunden ist, die ihrerseits in den Knochen in entsprechende Aussparungen eingebracht werden und eine feste Verbindung mit dem Knochen eingehen können.

Von diesem Stand der Technik geht die Erfindung gemäß dem Oberbegriff des Patentanspruches 1 aus.

Bei diesem bekannten System sind mindestens zwei unabhängige mit eigenen Verankerungsstiften versehene plattenartige Teilimplantate für wenigstens den teilweisen Ersatz der Knorpelschicht im betreffenden Gelenkbereich vorgesehen. Die Teilimplantate mit ihren Verankerungselementen werden in herkömmlicher Form aus Metall hergestellt und verbleiben nach dem Einsatz in den Knochen als Fremdkörper im Gelenkbereich, was bekanntermaßen zu Lockerungen und anderen Schädigungen führen kann.

Die WO98/56317 A offenbart eine Struktur zur Deckung von Knorpeldefekten, die aus einem gewickelten Gewebe besteht, demnach offenporig ist, und die aus einem biologisch abbaubaren Material gefertigt ist. Diese Knorpelersatzstruktur wird mit Befestigungsstiften in den Knochenteilen verankert, wobei diese Befestigungsstifte ebenfalls aus einem bioabsorbierbaren Material bestehen.

Aus der US-A-5067964 ist eine Knorpelersatzstruktur z. B. für ein Kniegelenk bekannt, die aus einer Vliesstruktur gebildet ist, die mit einer Abdeckung z. B. aus Polyurethan abgedeckt ist. Hiermit wird erreicht, dass die Ersatzstruktur flexibel bleibt und sich an die Gelenkstruktur anpassen kann. Die Abdeckung bildet eine glatte, jedoch feste Oberfläche auf der Ersatzstruktur.

Alle diese Dokumente liefern jedoch kein unmittelbar befriedigendes Ergebnis.

Es stellt sich daher erfindungsgemäß die Aufgabe, eine Knorpelersatzstruktur sicher und biologisch sinnvoll im Knochenlager zu verankern. Die Fixierung im Knochen soll einfach zu handhaben sein, größtmögliche mechanische Stabilität der Knorpelersatzstruktur auf dem Knochenlager gewährleisten und - je nach Ausführung der Erfindung - die Migration der Zellen aus dem Knochenmark in die Knorpelersatzstruktur nicht behindern.

Diese Aufgabe wird erfindungsgemäß durch die Merkmale des Anspruches 1 gelöst.

Weitere Ausgestaltungen der Erfindung gehen aus den Unteransprüchen hervor.

Demgemäss ist die Knorpelersatzstruktur eine durchgehend offenporige Struktur aus biologisch abbaubarem Material, sodass Zellen aus dem Knochen in die Knorpelersatzstruktur einwandern können.

Um das Einwandern der Zellen aus den Knochen in die Knorpelersatzstruktur zu erleichtern, können die Verankerungsstifte zum Beispiel in Längsrichtung eine Bohrung aufweisen.

Die Verankerungsstifte bestehen vorteilhaft aus einem bioresorbierbaren Material. Damit baut sich die gesamte Knochenersatzstruktur allmählich ab und wird durch die sich nachbildenden körpereigenen Zellen ersetzt. Eine solche Knorpelersatzstruktur kann dann ohne oder nach vorheriger Kultivierung mit Knorpel- oder Knorpelvorgängerzellen in das Knochenlager eingesetzt werden.

Somit sind gemäß der Erfindung die Verankerungsstifte fest mit der Knorpelersatzstruktur an ihrer dem Knochen zugewandten Seite verbunden. Dabei müssen die Verankerungsstifte auf der dem Knochen zugewandten Seite mit der Knorpelersatzstruktur verbunden sein, damit die dem Gelenk zugewandte Seite der Knorpelersatzstruktur nicht durch die Verankerungsstifte (z.B. Schraubenkopf, s.o.) unterbrochen wird. Dabei werden die Verankerungsstifte in den Knochen so eingebracht, dass sie dort vorzugsweise auf Grund von Reibung, Klemmung oder Formschluss eine feste Verbindung mit dem Knochen eingehen.

Die Form der Verankerungsstifte kann unterschiedlich gestaltet sein. So können die Verankerungsstifte über Widerhaken (9) verfügen und spitz zulaufend (8) gefertigt sein. Um das intraoperative Einbringen der Knorpelersatzstruktur zu erleichtern, kann es sinnvoll sein, wenn die Verankerungsstifte eine Vorrichtung aufweisen, mit der sie in einem entsprechenden Einbringwerkzeug (6) fixiert werden können. Dazu können die Verankerungsstifte z.B. Bohrungen (Sackloch- oder durchgehenden Bohrungen) (4) aufweisen. In einer weiteren Ausführungsform der Erfindung ist es auch denkbar, dass die Verankerungsstifte ein sternförmiges Profil mit mindestens drei Rippen (10) aufweisen, und dass das Einbringwerkzeug diese Sterne von außen her (11) umfaßt. Natürlich sind auch andere Ausgestaltungen der Einbringwerkzeuge denkbar, so insbesondere auch in sich geteilte Einbringwerkzeuge, die ein Einbringen einer Knorpelersatzstruktur aus verschiedenen Winkeln her erlauben.

Die Verbindung von Knorpelersatzstruktur und Verankerungsstiften kann z.B. eine Klebeverbindung sein, wobei als Kleber z.B. Polylactide, Polyglycolide o.ä. verwendet werden können, welche in einem geeigneten Lösungsmittel (Aceton, Chloroform o.ä.) aufgelöst wurden. Auch die Verklebung der Verankerungsstifte mit der Knorpelersatzstruktur mit einem solchen Lösungsmittel in purer Form ist denkbar. Bei dem Klebevorgang muss darauf geachtet werden, dass nur die dem Knochen zugewandte Seite des Vlieses und hier nur die Kontaktstellen zu den Verankerungsstiften verklebt werden, so dass die dem Gelenk zugewandte Schicht ihre Offenporigkeit behält. Auch eine thermische Verschmelzung von Knorpelersatzstruktur und Verankerungsstifte oder die direkte Verwebung der Verankerungsstifte mit der Knorpelersatzstruktur ist denkbar.

In einer besonderen Ausführungsform der Erfindung, insbesondere wenn die Knorpelersatzstruktur vorher mit Knorpel- oder Knorpelvorgängerzellen beladen werden soll, kann die dem Knochen, aber auch die dem Gelenk zugewandte Seite mit einer zusätzlichen Schicht, z.B. einer Membran versiegelt sein, da es denkbar ist, dass die ja bereits in diesen Fällen in der Knorpelersatzstruktur vorhandenen Zellen durch den Kontakt mit dem Knochenmilieu geschädigt werden.

Speziell in den Fällen, in denen ein Umdifferenzierung der aus dem Knochen in die Knorpelersatzstruktur einwandernden Zellen erfolgen soll, ist eine Versiegelung der dem Knochen zugewandten Seite allerdings nicht sinnvoll. Hier ist es im Gegenteil von Vorteil, wenn die Knorpelersatzstruktur auf der dem Knochen zugewandten Seite so offenporig wie möglich ist und die Knochenoberfläche selbst an möglichst vielen Stellen eröffnet wird. Dabei darf allerdings die Kontur der Knochenoberfläche nicht so weit zerstört werden, dass danach die Form der Gelenkoberfläche nach Auflage der Knorpelersatzstruktur nicht mehr gewährleistet ist. Besonders vorteilhaft ist es in diesen Fällen auch, wenn die Verankerungsstifte eine durchgehende Hohlbohrung (4) aufweisen, durch die ebenfalls Zellen aus dem Knochen in die Knorpelersatzstruktur einwandern können.

Die Zubereitung des Knochens kann z.B. mit einem speziellen Knochenbearbeitungsinstrument (12) erfolgen, welches auch die Ausnehmungen für die Verankerungselemente vorbereitet.

In einer speziellen Ausführungsform der Erfindung ist ***nur*** die dem Gelenk zugewandte Seite mit einer zusätzlichen Schicht, z.B. einer Membran, abgedeckt oder - z.B. durch thermische Behandlung - versiegelt ("Gelenkabschlussschicht") (3). Dieses ist insbesondere aus tribologischen Gründen wichtig, wenn einander gegenüber eingebrachte Knorpelersatzstrukturen gegeneinander laufen sollen. Auch kann ein Verlust von Zellen aus der Knorpelersatzstruktur in das Gelenk hinein durch eine solche Schicht verhindert werden.

In einer besonderen Ausführungsform der Erfindung kann die Anheftung einer Knorpelersatzstruktur auf einer Knochenoberfläche auch mit krampen-, stift- oder schraubenartig gestalteten Verankerungselementen gestaltet sein, die zunächst ***nicht*** fest mit dem Vlies, d. h. der Knorpelersatzstruktur, verbunden sind, sondern erst mit dem Vlies während der Operation fest verbunden werden, wonach das Vlies an den Knochen angeheftet wird. Auf der dem Gelenk zugewandten Seite müssen diese Verankerungselemente aber dann mit dem gleichen oder einem vergleichbaren Material abgedeckt sein, aus dem die Knorpelersatzstruktur besteht, so dass die harte Oberfläche der Verankerungselemente zum Gelenk hin abgedeckt ist. Der dabei zwangsweise entstehenden Stufe kann durch eine entsprechend dünne Abdeckschicht der Verankerungselemente entgegen gewirkt werden. Ein mit dem Vlies nicht fest verbundenes Verankerungselement, welches dadurch gekennzeichnet ist, dass es auf der dem Gelenk zugewandten Seite mit dem gleichen oder einem vergleichbaren Material abgedeckt ist, aus dem die Knorpelersatzstruktur besteht, ist jedoch nicht Gegenstand der Erfindung. Ein spezieller Einschläger (19), der die Aufnahme von mindestens einem Verankerungselement erlaubt, erleichtert das Einbringen der Verankerungselemente in den Knochen.

In einer besonderen Ausführungsform der Erfindung kann die Knorpelersatzstruktur zu besseren Anpassung an die vorherrschende Gelenkgeometrie dieser bereits von vornherein angepasst sein, was insbesondere bei größeren Knorpeldefekten wichtig ist. Die Daten zur Anpassung der Knorpelersatzstrukturgeometrie an die vorherrschenden Gelenkgeometrie können dabei aus bildgebenden Daten (z.B. CT, MRT) gewonnen werden, so dass auch individuell an den Patienten angepasste Knorpelersatzstrukturen hergestellt werden können. Diese Daten können dann auch als Basis für eine robotergestützte Zubereitung des Knorpel - und Knochenlagers dienen.

Als Materialien einer Knorpelersatzstruktur, der Verankerungsstifte sowie der die Knorpelersatzstruktur abschließenden Membranen kommen biologisch abbaubare (resorbierbare) Materialien in Betracht, wie sie für vergleichbare Anwendungen bereits im Einsatz sind. Insbesondere kommen z.B. hydrolysierbare Polymere der α-und β-Hydorxycarbonsäuren in Betracht. Typische Beispiele sind Polymere auf Basis der Polyglykolsäure, Polymere der Glykolsäure (PGA), Polylactide, Polymere von L-Lactid (P-L-LA), D-Lactid (P-D-LA), DL-Lactid (P-DL-LA), Poldioxanon, Polycaprolacton, Copolymere und Terpolymere aus den genannten Monomeren und/oder Mischungen aus den genannten Polymeren. Vorteilhaft können auch Copolymere von Glykolsäure und Lactid im Verhältnis von 99:1 bis 1:99, insbesondere im Verhältnis 89:11 bis 70:30 oder von 30:70 bis 11:89 eingesetzt werden. Ebenfalls von Vorteil können Copolymere von L-Lactid und DL-Lactid im Verhältnis 99:1 bis1:99, insbesondere im Verhältnis 99:1 bis 70:30 sein. Die verwendeten Substanzen können ggf. auch mit weichmachenden Zusätzen wie Caprolacton, Trimethylencarbonat, Triethylcitrat und/oder Acetylbutylcitrat versetzt werden. Auch Matrixstrukturen aus Kollagenen, insbesondere Kollagen I, Kollagen II und Kollagen III, auch in Kombination der einzelnen oder aller Partner zusammen, kommen in Frage.

Es kann von Vorteil sein, die Polymere für die Gelenkabschlussschicht sowie für die Schicht zwischen Knorpelersatzstruktur und Knochen verwendeten Polymere aus Lösung zu verarbeiten. Es ist auch möglich, fertige Membranen thermoplastisch oder mit geeigneten Klebern mit der Knorpelersatzstruktur zu verbinden. Auch Gelatine ist als Material derartiger Schichten denkbar, wobei die Wasserstabilität der Gelatine mit entsprechenden Zusätzen verbessert werden kann.

Die offenporige Knorpelersatzstruktur kann im Sinne eines Vlieses ausgeführt sein. Die Filamentstärke eines solchen Vlieses liegt dabei vorteilhaft zwischen 1 bis 50µm. Grundsätzlich sind aber auch andere Strukturen denkbar, z.B. auch Schäume, sofern sie eine offene Porosität aufweisen. Auch Strukturen, die einen regelhaften Aufbau wie in DE 197 21 661 A1 besitzen, sind gut geeignet.

Bei den Verankerungsstiften können auch nicht resorbierbare Materialien wie Knochenersatzmaterialien z.B. auf Hydroxylapatitbasis eingesetzt werden. Es ist auch denkbar, dem resorbierbaren Material (s.o.) der Verankerungselemente Zusätze wie Hydroxylapatit, Tricalciumphosphat, Misch-Calciumphosphate und/oder Calciumcarbonat sowie Wachstumsfaktoren beizufügen.

In einer besonderen Ausführungsform der Erfindung kann sowohl der Knorpelersatzstruktur als auch den Verankerungselementen Wachstumsfaktoren wie BMP (auch als recombinant growth factor), TgF-α sowie o.ä. TgF-β, IGF (insulin-like growth factor) osteogenes Protein, BFGF (basic fibroblast growth factor) und EGF (endothelial growth factor) mit beigesetzt werden. Die Konzentrationen dieser Faktoren sollte im Bereich von 0.1 bis 50 ng/ml liegen. Aber auch andere Wachstumsfaktoren sind denkbar.

Der Erfindung, das Umfeld der Einbringung der Knorpelersatzstrukturen sowie die schonende und die Kontur der Knochenoberfläche erhaltende Vorbereitung des Knochenlagers wird in den Zeichnungen anhand einiger bevorzugter Ausführungsbeispiele näher erläutert.

Dabei zeigt die Fig. 1 eine Knorpelersatzstruktur mit Verankerungsstiften und Einbringwerkzeug, Fig. 2 eine Knorpelersatzstruktur mit speziellen Verankerungsstiften, Fig. 3 eine weitere Ausführungsform spezieller Verankerungsstifte, Fig. 4 ein Spezialwerkzeug (12) zum Vorbereiten des Knochenlagers und Fig. 5 spezielle Verankerungselemente mit entsprechendem Einbringwerkzeug. Im Einzelnen zeigt die:

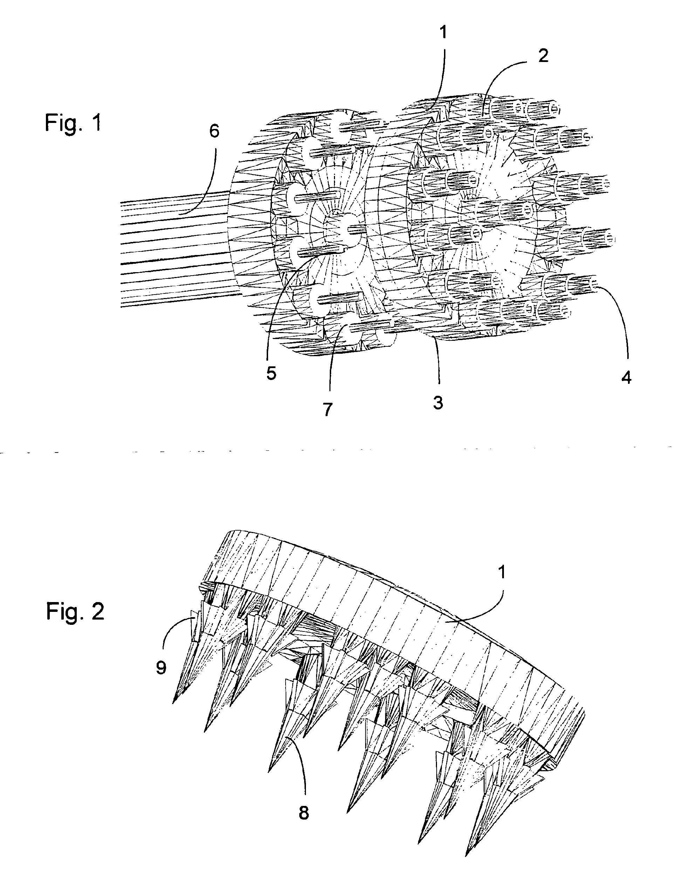

- Fig. 1: eine Knorpelersatzstruktur (1) mit damit fest verbun- denen Verankerungsstiften (2) als Verankerungselemen- te sowie einer Gelenkabschlussschicht (3). Die Veran- kerungsstifte sind mit zentralen Hohlbohrungen (4) versehen, in die auch die Zentrierstifte (5) des Ein- schlägers (6) eingreifen. In diesem Ausführungsbei- spiel wurde von einer kugeligen Gelenkoberfläche aus- gegangen. Die Knorpelersatzstruktur und die Gelenkab- schlussschicht sind in diesem Ausführungsbeispiel ei- ner kugeligen Oberfläche dieser Gelenkgeometrie be- reits von vornherein angepasst. In diesem Ausfüh- rungsbeispiel verfügt das Einbringwerkzeug über Erhö- hungen (7) im Bereich der Zentrierstifte, die dafür Sorge tragen dass die Verankerungsstifte (2) trotz der federnden Konsistenz der Knorpelersatzstruktur weit genug eingeschlagen werden können.
- Fig. 2: eine Knorpelersatzstruktur (1) mit damit fest verbun- denen speziellen spitz zulaufenden Verankerungsstif- ten (8) als Verankerungselemente. Die Verankerungs- stifte sind mit Widerhaken (9) versehen, um die Haf- tung im Knochenlager zu verbessern. Keine Gelenkab- schlussschicht in diesem Ausführungsbeispiel.

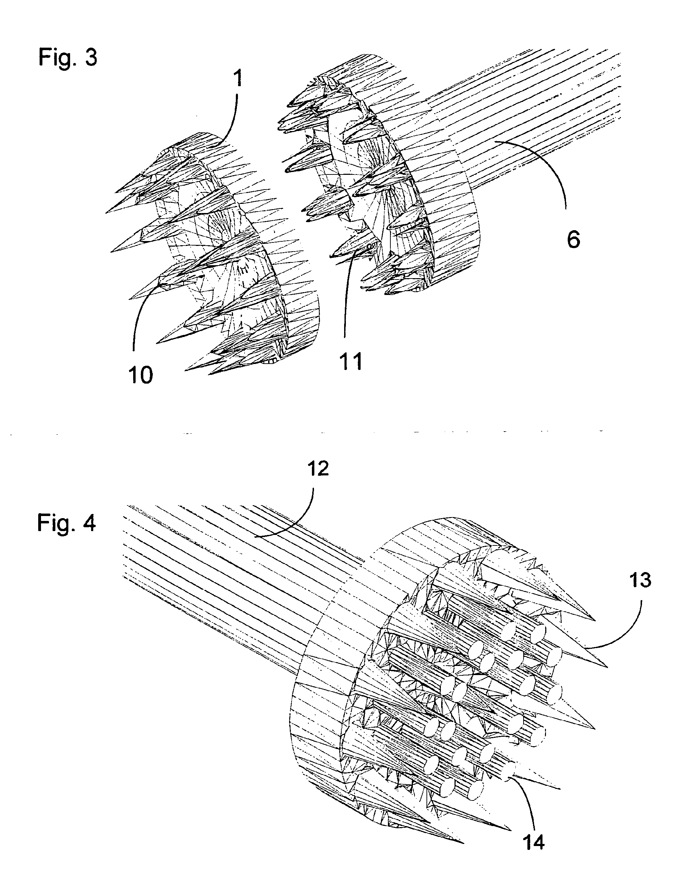

- Fig. 3: eine Knorpelersatzstruktur (1) mit damit fest verbun- denen speziellen Verankerungsstiften (10) als Veran- kerungselemente. Die Verankerungsstifte (10) verfügen über ein sternförmiges Profil und werden von außen von den Halterungselementen (11) des Einbringwerkzeu- ges (6) umfasst. Keine Gelenkabschlussschicht in die- sem Ausführungsbeispiel.
- Fig. 4: ein Spezialwerkzeug (12) zum Vorbereiten des Knochen- lagers. Nach Entfernen des Knorpel werden die Zapfen (13,14) des Spezialwerkzeuges (12) in den Knochen eingeschlagen. Dabei eröffnen die speziellen Zapfen (13) den Knochen zur Aufnahme der Verankerungselemen- te, die übrigen Zapfen (14) sorgen für weitere punk- tuelle Stanzdefekte im Knochen, aus denen Zellen in das Vlies migrieren können, ohne das die Kontur der Knochenoberfläche verloren geht.

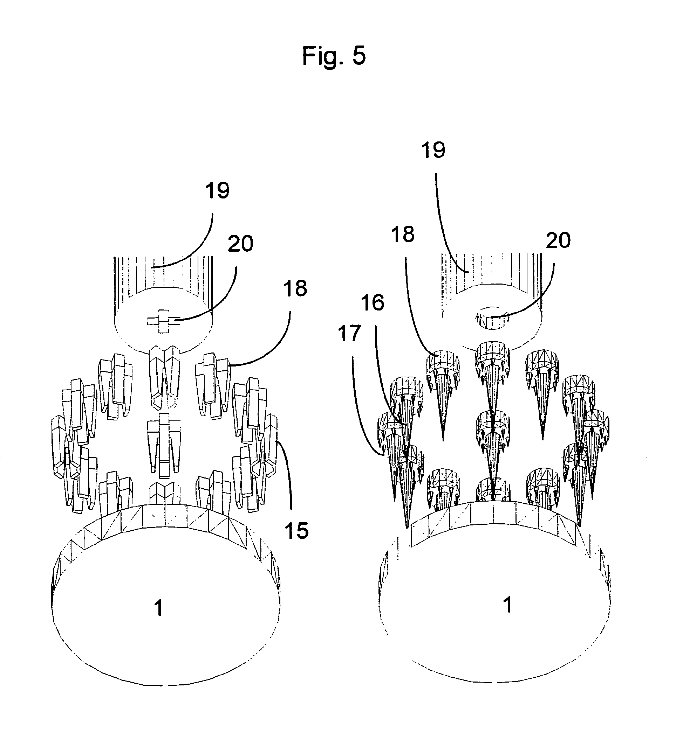

- Fig. 5: eine Verankerung der Knorpelersatzstruktur (1) mit- tels krampenartiger (15) oder stiftartiger (16) Ver- ankerungselemente, mit welchen erst während der Ope- ration eine Verbindung der Verankerungselemente mit der Knorpelersatzstruktur und schließlich eine Anhef- tung der Knorpelersatzstruktur an den Knochen erzielt wird. Die Verankerungselemente sind mit einer der Knorpelersatzstruktur ähnlichen Schicht (18) abge- deckt. Im dargestellten Ausführungsbeispiel fehlt ei- ne Gelenkabschlussschicht sowohl auf der Knorpeler- satzstruktur (1) als auch auf den Verankerungselemen- ten, welches natürlich prinzipiell ebenso wie eine weitere Schicht im Sinne einer Membran zwischen Kno- chen und Knorpelersatzstruktur möglich ist. An dem stiftartigen Verankerungselement sind in dem darge- stellten Ausführungsbeispiel auch noch kleine Stift- chen (17) angebracht, um ein sicheres Fixieren des Vlieses zu ermöglichen. Die Stifte können in einem Einschläger (19) mit entsprechender Aussparung (20) zum Einschlagen in den Knochen gefasst werden.

## Patentansprüche

1. Struktur zur Deckung von Knorpeldefekten, die im Bereich des Knorpeldefektes einsetzbar ist und an ihrer dem Knochen zugewandten Seite fest mit Verankerungsstiften (2, 8, 10) versehen ist, die ihrerseits in den Knochen in entsprechende Aussparungen einbringbar und dort fest in dem Knochen verankerbar sind, wobei die Struktur eine Knorpelersatzstruktur (1) aus biologisch abbaubarem Material aufweist, **dadurch gekennzeichnet, dass** die Knorpelersatzstruktur (1) offenporig ausgebildet ist, sodass aus dem Knochen Zellen in die Knorpelersatzstruktur (1) wandern können.

2. Struktur zur Deckung von Knorpeldefekten nach Anspruch 1, **dadurch gekennzeichnet, dass** die Verankerungsstifte (10) aus biologisch abbaubarem Material bestehen.

3. Struktur zur Deckung von Knorpeldefekten nach Anspruch 1, **dadurch gekennzeichnet, dass** die feste Verbindung zwischen Verankerungsstiften (2) und Knochen aufgrund von Reibung, aufgrund von Klemmung und/oder aufgrund eines Formschlusses erfolgt.

4. Struktur zur Deckung von Knorpeldefekten nach einem der vorhergehenden Ansprüche, **dadurch gekennzeichnet, dass** unter der Knorpelersatzstruktur (1) zum Knochen hin eine Schicht (3) zur Abdeckelung der Knorpelersatzstruktur (1) aufgebracht ist.

5. Struktur zur Deckung von Knorpeldefekten nach einem der vorhergehenden Ansprüche, **dadurch gekennzeichnet, dass** auf der Knorpelersatzstruktur (1) sowohl zum Gelenk hin als auch zum Knochen hin je eine Schicht (3) zur Abdeckelung der Knorpelersatzstruktur aufgebracht ist.

6. Struktur zur Deckung von Knorpeldefekten nach einem der vorhergehenden Ansprüche, **dadurch gekennzeichnet, dass** die Knorpelersatzstruktur (1) an die äußere Geometrie der zu ersetzenden Knorpelschicht angepasst ist, wobei Standardgeometrien wie z.B. Kugeloberflächen verschiedener Durchmesser oder aber individuelle Gelenkoberflächen aus 3D-Rekonstruktionen z.B. aus CT-Datensätzen oder ähnlichen bildgebenden Verfahren verwendet werden können.

7. Struktur zur Deckung von Knorpeldefekten nach einem der vorhergehenden Ansprüche, **dadurch gekennzeichnet, dass** in der Knorpelersatzstruktur (1) Wachstumsfaktoren mit beigesetzt werden.

8. Struktur zur Deckung von Knorpeldefekten nach einem der vorhergehenden Ansprüche, **dadurch gekennzeichnet, dass** die zur Anheftung der Knorpelersatzstruktur(1) auf der Knochenoberfläche verwendeten und mit der Knorpelersatzstruktur fest verbundenen Verankerungsstifte (15, 16) auf der dem Gelenk hin zugewandten Seite über einen der Knorpelersatzstruktur entsprechenden Aufbau verfügen, so dass die dem Gelenk zugewandte Seite der Verankerungsstifte zum Gelenk hin abgedeckt ist (bei 18).

9. Struktur zur Deckung von Knorpeldefekten nach einem der vorhergehenden Ansprüche, **dadurch gekennzeichnet, dass** die Verankerungsstifte (2) in Längsrichtung eine Bohrung (4) aufweisen.

10. Struktur zur Deckung von Knorpeldefekten nach einem der vorhergehenden Ansprüche, **dadurch gekennzeichnet, dass** die Verankerungsstifte (8) mit Widerhaken (9) versehen sind.

11. Struktur zur Deckung von Knorpeldefekten nach einem der vorhergehenden Ansprüche, **dadurch gekennzeichnet, dass** der Querschnitt der Verankerungsstifte ein Sternprofil mit mindestens drei Rippen aufweist.

12. Struktur zur Deckung von Knorpeldefekten nach einem der vorhergehenden Ansprüche, **dadurch gekennzeichnet, dass** den Verankerungsstiften aus resorbierbarem Material Zusätze wie Hydroxylapatit, Tricalciumphosphat, Misch-Calciumphosphate und/oder Calciumcarbonat sowie Wachstumsfaktoren beigegeben sind.

13. Struktur zur Deckung von Knorpeldefekten nach einem der vorhergehenden Ansprüche, **dadurch gekennzeichnet, dass** die Verankerungsstifte aus Knochenersatzmaterialien hergestellt sind.

## Claims

1. A system for correcting cartilage defects, which can be used in the area of the cartilage defect and is provided with fixed anchoring pins (2, 8, 10) on its side facing the bone, which, in turn, can be inserted into the bone into corresponding recesses and can be fixedly anchored there in the bone, the structure having a substitute cartilage structure (1) from biologically degradable material, **characterized in that** the substitute cartilage structure (1) is made with open pores so that cells can migrate from the bone into the substitute cartilage structure (1).

2. The structure for correcting cartilage defects according to claim 1, **characterized in that** the anchoring pins (10) consist of a biologically degradable material.

3. The structure for correcting cartilage defects according to claim 1, **characterized in that** the fixed connection between anchoring pins (2) and bone is produced based on friction, based on clamping and/or based on a positive fit.

4. The structure for correcting cartilage defects according to any of the preceding claims, **characterized in that** a layer (3) for covering the substitute cartilage structure (1) is applied below the substitute cartilage structure (1) towards the bone.

5. The structure for correcting cartilage defects according to any of the preceding claims, **characterized in that** one layer (3) each is applied on the substitute cartilage structure (1) both towards the joint and towards the bone to cover the substitute cartilage structure.

6. The structure for correcting cartilage defects according to any of the preceding claims, **characterized in that** substitute cartilage structure (1) is adapted to the outer geometry of the cartilage layer to be replaced, standard geometries such as spherical surfaces of various diameters or individual joint surfaces from 3D reconstructions, e.g. from CT data sets or similar imaging procedures, may be used.

7. The structure for correcting cartilage defects according to any of the preceding claims, **characterized in that** growth factors are added to the substitute cartilage structure (1).

8. The structure for correcting cartilage defects according to any of the preceding claims, **characterized in that** the anchoring pins (15, 16) which are used for affixing the substitute cartilage structure (1) on the bone surface and are fixedly connected with the substitute cartilage structure have a structure corresponding to the substitute cartilage structure on the side facing the joint so that the side of the anchoring elements, which face the joint, is covered towards the joint (at 18).

9. The structure for correcting cartilage defects according to any of the preceding claims, **characterized in that** the anchoring pins (2) have a bore (4) in the longitudinal direction.

10. The structure for correcting cartilage defects according to any of the preceding claims, **characterized in that** the anchoring pins (8) are provided with barbs (9).

11. The structure for correcting cartilage defects according to any of the preceding claims, **characterized in that** the cross-section of the anchoring pins has a star profile with at least three ribs.

12. The structure for correcting cartilage defects according to any of the preceding claims, **characterized in that** additives such as hydroxyl apatite, tricalcium phosphate, mixed calcium phosphates and/or calcium carbonate and growth factors are added to the anchoring pins made of a resorbable material.

13. The structure for correcting cartilage defects according to any of the preceding claims, **characterized in that** the anchoring pins are produced from substitute cartilage structure bone materials.

## Revendications

1. Structure pour le recouvrement de défauts de cartilage, qui peut être mise en place dans la zone du défaut de cartilage et qui est pourvue, sur sa face tournée vers l'os, de pointes d'ancrage (2, 8, 10) de manière solidaire qui peuvent, de leur part, être insérées dans l'os dans des évidements correspondants et y être ancrées de manière solidaire dans l'os, la structure présentant une structure de substitution de cartilage (1) en matériau biodégradable, **caractérisée en ce que** la structure de substitution de cartilage (1) est réalisée à pores ouverts, de telle sorte que depuis l'os, des cellules peuvent migrer dans la structure de substitution de cartilage (1).

2. Structure pour le recouvrement de défauts de cartilage selon la revendication 1, **caractérisé en ce que** les pointes d'ancrage (10) sont en matériau biodégradable.

3. Structure pour le recouvrement de défauts de cartilage selon la revendication 1, **caractérisée en ce que** la liaison solidaire entre les pointes d'ancrage (2) et l'os est produite par friction, par serrage et/ou par une fixation par coopération de formes.

4. Structure pour le recouvrement de défauts de cartilage selon l'une quelconque des revendications précédentes, **caractérisée en ce qu'**une couche (3) est appliquée sous la structure de substitution de cartilage (1) vers l'os pour recouvrir la structure de substitution de cartilage (1).

5. Structure pour le recouvrement de défauts de cartilage selon l'une quelconque des revendications précédentes, **caractérisée en ce qu'**une couche (3) respective est appliquée sur la structure de substitution de cartilage (1) tant vers l'articulation que vers l'os pour recouvrir la structure de substitution de cartilage.

6. Structure pour le recouvrement de défauts de cartilage selon l'une quelconque des revendications précédentes, **caractérisée en ce que** la structure de substitution de cartilage (1) est adaptée à la géométrie extérieure de la couche de cartilage à substituer, des géométries standard telles que par exemple des surfaces sphériques de différents diamètres ou aussi des surfaces d'articulation individuelles à partir de reconstructions 3D, par exemple à partir de jeux de données de CT ou de procédés d'imagerie similaires.

7. Structure pour le recouvrement de défauts de cartilage selon l'une quelconque des revendications précédentes, **caractérisée en ce que** des facteurs de croissance sont ajoutés dans la structure de substitution de cartilage (1).

8. Structure pour le recouvrement de défauts de cartilage selon l'une quelconque des revendications précédentes, **caractérisée en ce que** les pointes d'ancrage (15, 16) utilisées pour fixer la structure de substitution de cartilage (1) sur la surface de l'os et reliées de manière solidaire à la structure de substitution de cartilage sur la face tournée vers l'articulation disposent d'une constitution similaire à la structure de substitution de cartilage, de telle sorte que la face des pointes d'ancrage qui est tournée vers l'articulation est recouverte vers l'articulation (sur 18).

9. Structure pour le recouvrement de défauts de cartilage selon l'une quelconque des revendications précédentes, **caractérisée en ce que** les pointes d'ancrage (2) présentent un perçage (4) en direction longitudinale.

10. Structure pour le recouvrement de défauts de cartilage selon l'une quelconque des revendications précédentes, **caractérisée en ce que** les pointes d'ancrage (8) sont pourvues d'ardillons (9).

11. Structure pour le recouvrement de défauts de cartilage selon l'une quelconque des revendications précédentes, **caractérisée en ce que** la section transversale des pointes d'ancrage présente un profil en étoile avec au moins trois nervures.

12. Structure pour le recouvrement de défauts de cartilage selon l'une quelconque des revendications précédentes, **caractérisée en ce qu'**il est ajouté aux pointes d'ancrage en matériau résorbable des additifs tels que l'hydroxylapatite, le phosphate de tricalcium, des phosphates de calcium mélangés et/ou du carbonate de calcium ainsi que des facteurs de croissance.

13. Structure pour le recouvrement de défauts de cartilage selon l'une quelconque des revendications précédentes, **caractérisée en ce que** les pointes d'ancrage sont réalisées en matériaux de substitution osseuse.
